# EUROPEAN PATENT APPLICATION

(11) **EP 0 791 659 A2**
(43) Date of publication of application: **27.08.1997**
(21) Application number: 97200874.2
(22) Date of filing: 13.11.1990
(51) Int. Cl.: C12Q 1/68, G01N 33/53, C12N 5/00

(54) **Antigen specific separation of nucleated fetal cells in non-invasive detection of fetal DNA**

(30) Priority: 13.11.1989 US 436057
(62) Divisional of application: 90917320.5
(71) Applicant: CHILDREN'S MEDICAL CENTER CORPORATION, Boston Massachusetts 02115 (US)
(72) Inventor: Bianchi, Diana W., Brookline, Massachusetts 02146 (US)
(74) Representative: Holdcroft, James Gerald, Dr.

(57) **Abstract**

A method of isolating fetal nucleated cells, particularly fetal nucleated erythrocytes, from a maternal blood sample, by means of an antigen present on the cell surface of the fetal erythrocytes. A method of detecting fetal DNA of interest, which is a gene or gene portion associated with a disease or condition, a chromosomal abnormality or sex-specific DNA, in a maternal blood sample. The presence or absence, as well as the quantity of fetal DNA of interest in a maternal sample can be determined. The claimed method of detection can be used prenatally or postnatally and is particularly useful because it is non-invasive and can be carried out early in pregancy.

## Description

### Funding

Work described herein was supported by the National Institutes of Health and Children's Hospital Medical Center.

### Background

A variety of fetal cell types--platelets, trophoblasts, erythrocytes and leucocytes--cross the placenta and circulate transiently within maternal blood (Schroder, J., J. Med. Genet., 12:230-242 (1975); Douglas G.W. et al., Am. J. Obstet. Gynec., 78:960-973 (1959)). There have been numerous reports of efforts to separate fetal cells from maternal cells present in maternal blood, but none has been successful in isolating cells subsequently shown to contain fetal DNA. Distinguishing fetal cells from maternal cells has not been successful for several reasons, including the small number of fetal cells in a maternal blood sample and the fact that morphological differences are slight (e.g., trophoblasts are the only fetal cells which can be distinguished from maternal cells by morphology alone).

Others report screening the peripheral blood of pregnant women for cells of fetal origin. Fetal identification relied on the presence of a single cytogenetic marker, the Y chromosome. Lymphocytes with a putative "XY" karyotype were found in the maternal circulation as early as 14 weeks gestation (Walknowska, J., et al., The Lancet, 1119-1122 (1979)).

The availability of flow cytometry has led many to suggest that fetal cells could be obtained through the use of a flow cytometer and that such cells could be exploited for prenatal genetic diagnosis. However, although cells sorted in this manner have been said to be of fetal origin, based on analysis of cell surface antigens, morphology, or cytogenetic criteria, there has not been confirmation that the cells contain fetal DNA. A method by which fetal DNA could be obtained from maternal blood during pregnancy would be valuable, particularly if it made it possible to carry out prenatal diagnosis by a noninvasive technique.

### Disclosure of the Invention

The present invention is an in vitro method of separating or isolating fetal DNA present in the blood of a pregnant woman from maternal DNA, as well as an in vitro method of detecting the presence of and/or quantitating selected fetal DNA in fetal DNA, which is useful as a noninvasive prenatal diagnostic or analytical method.

In the present method, fetal nucleated cells are isolated from a maternal blood sample by means of a detectable material which binds to the fetal nucleated cells but not to maternal cells and is then separated from the maternal sample, resulting in separation of the fetal nucleated cells from the sample. The fetal nucleated cells can be any undifferentiated hematopoietic cell and, particularly, fetal nucleated erythrocytes. In one embodiment of the present method of isolation, at least one detectably labelled monoclonal antibody specific for an antigen present on fetal nucleated cells, but not for an antigen present on maternal cells, is combined with a maternal blood sample and, once bound to fetal nucleated cells, is separated from the maternal sample. Alternatively, at least one detectably labelled monoclonal antibody specific for an antigen present on maternal cells, but not for an antigen present on fetal nucleated cells is used. In a further embodiment, the two types of monoclonal antibodies are used.

In the case in which the detectable label is a fluorescent molecule, separation is carried out by means of flow cytometry, in which fluorescently-labelled molecules are separated from unlabelled molecules. This results in separation of fetal nucleated cells, such as fetal nucleated erythrocytes, from maternal cells and, thus, of fetal DNA from maternal DNA. That this separation has occurred can be verified using known techniques, such as microscopy or detection of fetal hemoglobin.

In one embodiment of the method of the present invention by which the occurrence of a selected DNA sequence or sequences (gene(s) or gene portion(s)) in fetal DNA is determined (detected and/or quantitated), the isolated fetal nucleated cells, such as fetal nucleated erythrocytes, are treated to render DNA present in them available for amplification. Amplification of DNA from fetal nucleated cells (fetal DNA) is carried out using a known amplification technique, such as the polymerase chain reaction (PCR). Amplified fetal nucleated cell DNA is subsequently separated on the basis of size (e.g., by gel electrophoresis) and contacted with a selected labelled probe, such as labelled DNA complementary to a selected DNA sequence (e.g., complementary to an abnormal gene or gene portion, or Y-specific DNA). Detection of the labelled probe after it has hybridized to fetal DNA results in detection of the sequence of interest in the fetal DNA. Quantitation of the hybridized labelled probe results in quantitation of the fetal DNA.

In a second embodiment of the present method of determining the occurrence of a selected DNA sequence (or sequences), cells isolated as described above are sorted onto a solid support, such as a slide, and screened for chromosomal abnormalities using in situ hybridization. In this embodiment, a selected nucleic acid probe, such as a labelled DNA probe for chromosomal DNA associated with a congenital abnormality, is combined with the fetal DNA, under conditions appropriate for hybridization of complementary sequences to occur. Detection and/or quantitation of the labelled probe after hybridization results in detection and/or quantitation of the fetal DNA to which the probe has hybridized.

The present method of detecting the occurrence of selected fetal DNA is useful for prenatal evaluation or diagnostic purposes, such as determination of the sex of the fetus, assessment of chromosomal abnormalities and determination of the presence of abnormal genes associated with human disease.

A particular advantage of the method of the present invention by which fetal nucleated erythrocytes are isolated is that such cells can be reliably separated from cells of maternal origin. In addition, because such cells are nucleated and, thus, contain a full complement of fetal genes, the present method makes available complete fetal DNA. The present method of detecting and/or quantitating a selected fetal DNA sequence is particularly valuable not only because of the advantages associated with the present method of isolating fetal cells, but also because it is a noninvasive technique which can be applied early in gestation.

### Brief Description of the Drawings

Figure 1 is a schematic representation of the method of the present invention by which fetal nucleated cells are isolated from maternal cells and DNA within the fetal cells is assessed for the occurrence of a particular fetal DNA sequence.

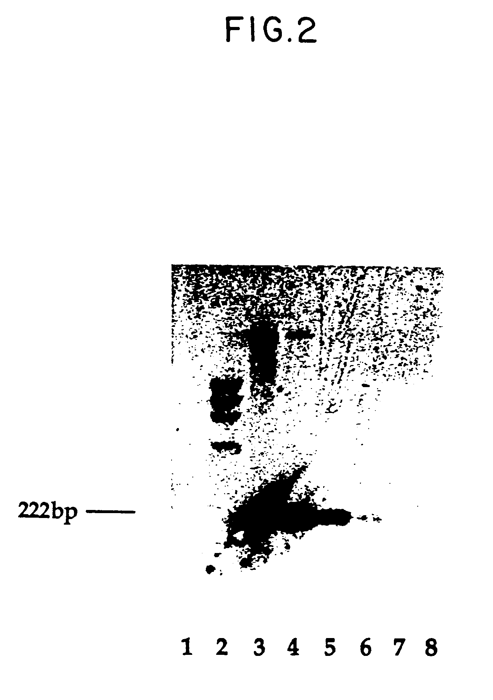

Figure 2 is an autoradiograph of diluted male DNA amplified for 222 bp sequence. Lane 1: reagent control; lane 2: φX174 molecular weight standard; lane 3: 100 ng; lane 4: 10 ng; lane 5: 1 ng; lane 6: 200 pcg.; lane 7: 10 pcg; lane 8: 1 pcg.

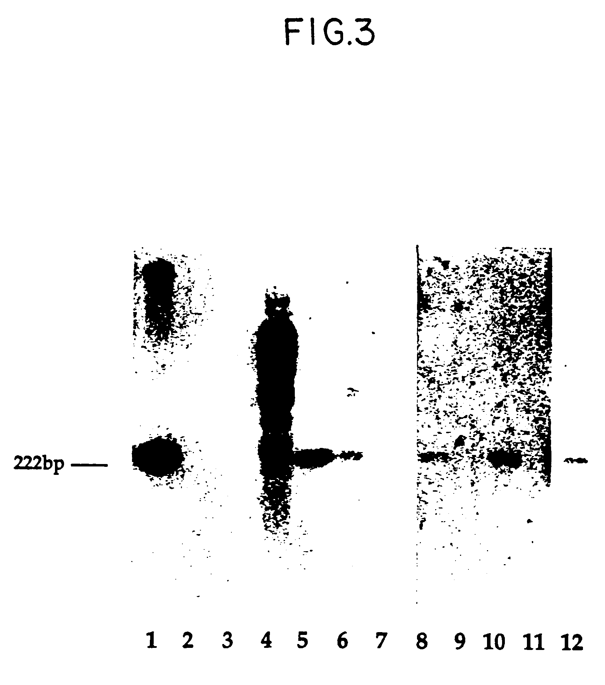

Figure 3 is a composite autoradiograph of amplified patient DNA. Lane 1: 10 ng normal male; lane 2: 10 ng normal female; lane 3: reagent control; lane 4: φX174; land 5: sorted cells from patient 1 (male fetus); lane 6: sorted cells from patient 2 (male fetus); lane 7: sorted cells from patient 3 (female fetus); lane 8: sorted cells from patient 6 (female fetus); lane 9: sorted cells from patient 7 (male fetus); lane 10: sorted cells from patient 8 (male fetus); lane 11: sorted cells from patient 9 (female fetus); lane 12: cord blood from female infant whose cells were prenatally sorted in lane 8.

Figure 4 is a diagram demonstrating the detection of Y chromosomal DNA sequences at various points of gestation in women bearing male pregnancies.

Figure 5 is a series of histograms (A through F) obtained when FITC-anti transferrin receptor was used to determine the presence of mononuclear cells in samples from non-pregnant females to which male cells have been added.

Figure 6 is a composite autoradiograph of amplified male DNA detected in TfR⁺ cells when 10²-10⁶ male cells are added to samples from non-pregnant females and in TfR⁻ cells when 10⁵-10⁶ male cells are added to samples from non-pregnant females.

Figure 7 is a series of histograms (A through H) obtained when anti HPCA-1 antibody was used to determine the presence of mononuclear cells in samples from non-pregnant females to which male cells have been added.

Figure 8 is a photograph illustrating a fluorescent cell due to the positive results of in situ hybridization of the pDP97 probe for the Y chromosome to a fetal nucleated red blood cell.

### Detailed Description of the Invention

The present invention relates to an in vitro method of separating or isolating fetal nucleated cells present in the blood of a pregnant woman (a maternal blood sample) from the pregnant woman's cells and of separating or isolating fetal DNA from maternal DNA. It further relates to an in vitro method of prenatal detection and/or quantitation of selected fetal DNA in fetal DNA isolated from the maternal blood sample. The method provides a noninvasive approach to detect and/or quantitate fetal DNA, such as that associated with a disease or a condition whose assessment during gestation is desired. It also provides a noninvasive means by which the sex of a fetus can be determined.

The following is a description of the basis for the subject method; of the present method of isolating nucleated fetal cells present in the blood of a pregnant woman from maternal cells and, subsequently, separating fetal DNA from maternal DNA; and of the present method of prenatal determination of the occurrence (presence/absence or quantitation) of selected DNA in fetal cells.

### Nucleated erythrocyte as a potential source of fetal genes

It has now been determined that fetal nucleated cells, present in the blood of a pregnant woman are a source of fetal genes. That is, it has been shown that fetal nucleated erythrocytes (also referred to as fetal NRBC) can be isolated or separated from maternal blood and that DNA present in the isolated fetal cells can be used to assess fetal characteristics (e.g., sex, presence or absence of chromosomal abnormalities).

Fetal nucleated erythrocytes were selected for sorting based on the following rationale:
1. In any given fetomaternal hemorrhage, no matter how small, the ratio of fetal erythrocytes to fetal lymphocytes should remain the same as in whole fetal blood; thus, there would be 1,000 times as many red cells as white cells available for analysis.
2. Normal pregnant females do not usually have circulating NRBC; therefore, an isolated NRBC would a priori have a greater chance of being fetal in origin.
3. The majority of pregnancies are blood group compatible, which means that the "transfused" NRBC would probably be tolerated by the mother and remain in her circulation.
4. Because they are nucleated, the NRBC contain a full complement of fetal genes.

### Advances in molecular biology applied to fetal cell sorting

Recent advances in molecular biology have had an enormous impact on the feasibility of fetal cell identification. For example, fluorescent in situ hybridization can be used for this purpose.

The development of the polymerase chain reaction (PCR) (Mullis, K., et al., Cold Spring Harb. Symp. Quant. Biol., 51:263-272 (1986)), with its capacity for DNA analysis from a single cell (Li, H., et al., Nature, 355:414-417 (1988); Handyside, A.H., et al., Lancet 1:347-349 (1989)), has eliminated the technical problems associated with the small number of fetal cells in maternal blood. It makes DNA diagnosis from a single cell possible.

As described below, fetal nucleated erythroblasts have been shown to be present in blood obtained from pregnant women, thus making maternal blood a useful/reliable potential source of fetal DNA; fetal nucleated cells have been distinguished from maternal cells on the basis of surface antigenic characteristics, thus making it possible to separate the two cell types from one another; and fetal DNA present in the separated fetal nucleated cells has been analyzed and characterized.

### Detection of fetal gene sequences in maternal blood

One of the first steps in developing the present method of isolating fetal nucleated cells from the maternal blood supply was identification of monoclonal antibodies that permit identification and separation of fetal cells from maternal cells present in blood obtained from a pregnant woman. This has been done, as described in detail in the Examples. As a result, it has been determined that monoclonal antibodies which recognize maternal leucocytes and monoclonal antibodies which recognize fetal cell surface antigens are useful in separating maternal and fetal cells. The following is a brief description of monoclonal antibodies which have been shown to be useful in separating fetal nucleated cells from maternal cells present in a maternal blood sample. However, other monoclonal antibodies which distinguish between fetal and maternal cells on the basis of surface antigenic differences, can also be used in the present method. The present method requires the use of at least one type of antibody which is specific for (or recognizes) a surface antigen present on fetal nucleated cells, for a surface antigen present on maternal cells, but not specific for both. That is, the present method can be carried out using one or more antibody which distinguishes fetal nucleated cells from maternal cells. The present method can be carried out using whole blood or blood treated or processed to enrich for (increase the concentration of) fetal nucleated cells.

Described below is the selection and successful use of monoclonal antibodies which distinguish fetal nucleated erythrocytes from maternal cells. It is to be understood, however, that in a similar manner, monoclonal antibodies which make it possible to select for another fetal nucleated cell type (or types) can be identified and used in the present method to separate fetal nucleated cells from maternal cells (and, thus, fetal DNA sources from maternal DNA).

Initial efforts focused on the elimination of contaminating maternal leucocytes in the mononuclear cell layer and identification of monoclonal antibodies effective in carrying out this separation, which results in production of a maternal sample enriched in fetal nucleated cells.

HLe-1 (Becton-Dickinson Monoclonal center, Mountain View, CA, catalog #7463) is a monoclonal antibody available as a direct fluorescein isothiocyanate (FITC) conjugate. It recognizes an antigen present on mature human leucocytes and on very immature erythrocyte precursors, but not on mature nucleated erythrocytes (Loken, M.E., et al., Blood, 69:255-263 (1987)). Thus, maternal leucocytes are recognized and bound, but fetal nucleated erythrocytes are not, making separation of the two possible. As described in detail in Example 1, this labelled antibody was used to eliminate maternal leucocytes in the mononuclear cell layer.

As is also described (Example 1), a combination of monoclonal antibodies has been used for the same purpose (i.e., elimination of maternal cells from the blood sample). As described, anti-monocyte antibody (M3) and anti-lymphocytes antibody (L4) have been used to remove maternal cells from the mononuclear cell layer resulting from density gradient centrifugation.

Monoclonal antibodies which recognize fetal nucleated cells but do not recognize maternal cells were also identified. As described in detail in Example 1, a monoclonal antibody which recognizes the transferrin receptor was identified. Erythroblasts have been shown to express the transferrin receptor (Loken, M.R., et al., Blood, 69:255-263 (1987)) antigen on their cell surfaces from the BFU-E stage until nuclear extrusion (Loken, M.R. et al., Blood, 69:255-263 (1987)). The transferrin receptor is also present on activated lymphocytes (Trowbridge, I.S. and M.B. Omary, Proc. Natl. Acad. Sci. USA, 78:3039-3043 (1981)), certain tumor cells (Greaves, M. et al., Int. J. Immunopharmac., 3:283-300 (1981)), and trophoblast cells (Galbraith, G.M.P. et al., Blood, 55:240-242 (1980)). Thus, such an antibody is specific for or recognizes (binds to) fetal nucleated cells, but not maternal leucocytes. As described in Example 1, commercially available fluorescein-conjugated monoclonal antibodies against the transferrin receptor (TfR) were used to separate fetal nucleated erythrocytes from maternal cells. Although the antibody is not specific for fetal nucleated erythrocytes, it facilitated their enrichment in the flow-sorted samples. Other monoclonal antibodies which are able to distinguish between fetal nucleated cells and maternal cells present in a blood sample can also be used. Such antibodies include commercially available monoclonal antibodies and those which can be produced using known techniques.

Separation of fetal nucleated cells from a maternal blood sample using antibodies described above can be carried out with samples of whole blood or a fraction of whole blood (i.e., one resulting from treatment or processing of whole blood to increase the proportion of fetal nucleated cells present, referred to as an enriched maternal sample. An enriched maternal sample is produced, for example, in a two-step process. The maternal sample is subjected to initial separation on the basis of size, such as by Ficoll-Hypaque density gradient centrifugation. This results in production of a supernatant layer, which contains platelets; a mononucluear cell layer; and an agglutinated pellet which contains non-nucleated erythrocytes and granulocytes. The mononuclear layer is separated from the other layers, to produce a maternal sample which is enriched in fetal nucleated cells.

The maternal sample, whether maternal whole blood or an enriched maternal sample, is subjected to separation, based on surface antigenic differences between fetal nucleated cells and maternal cells using antibodies described above. The maternal sample is contacted with at least one monoclonal antibody which is specific for either fetal nucleated cells or maternal cells, but not for both and, thus, makes it possible to separate the two types of cells. The maternal sample can be combined with a set of two or more monoclonal antibodies, each of which is specific for either fetal or maternal cells, but not for both. The combination of monoclonal antibodies can be designed to enhance separation of the two types of cells (e.g., the combination of anti-TfR antibody and HLe-1 antibody described previously) beyond that possible with a single monoclonal antibody. Separation of the fetal cells is carried out using known techniques, such as flow cytometry, use of immunomagnetic beads and cell panning. In general, the monoclonal antibodies have a detectable label (e.g., radioactive material, fluorophore).

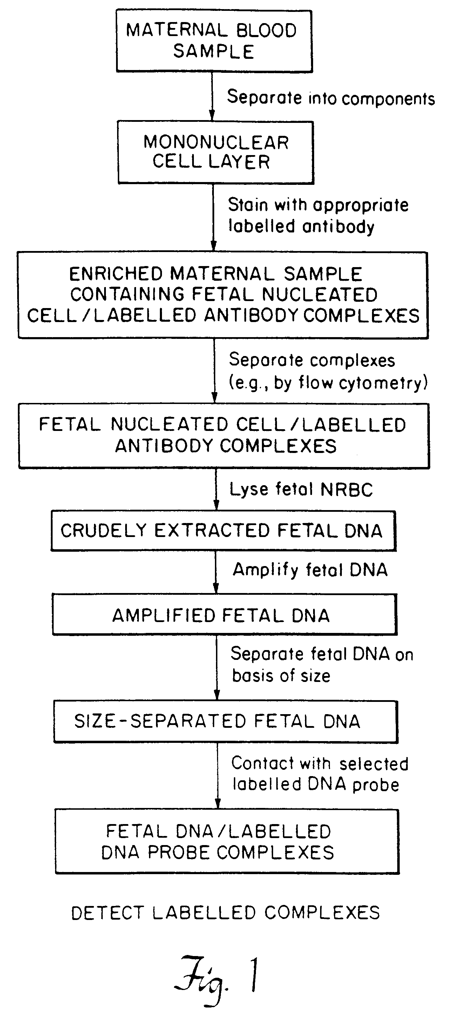

An embodiment of the method of the present invention by which fetal cells are isolated and fetal DNA is detected is represented schematically in Figure 1. A maternal blood sample (typically 20 ml.) is obtained, using known techniques. The sample is separated into component layers on the basis of size and the mononuclear cell layer, referred to as the maternal sample enriched in nucleated cells (or enriched maternal sample), is removed for further processing. The enriched maternal sample is contacted with at least one monoclonal antibody, as described above, and the resulting fetal nucleated cell/antibody complexes are separated using known methods (e.g., flow cytometry, immunomagnetic beads, cell panning). Fetal DNA is crudely extracted from the resulting complexes (e.g., by heat), thus rendering it available for hybridization with nucleic acid probes. Fetal DNA can be analyzed for a selected DNA sequence or DNA sequences, using known techniques. Prior to analysis, fetal DNA can be amplified, as needed, using known methods (e.g., PCR).

If amplification is to be carried out, the sorted samples are amplified for an appropriate number of cycles of denaturation and annealing (e.g., approximately 25-60). Control samples include a tube without added DNA to monitor for false positive amplification. With proper modification of PCR conditions, more than one separate fetal gene can be amplified simultaneously. This technique, known as "multiplex" amplification, has been used with six sets of primers in the diagnosis of DMD (Chamberlain, J.S., et al., Prenat. Diagnosis, 9:349-355 (1989)). When amplification is carried out, the resulting amplification product is a mixture which contains amplified fetal DNA of interest (i.e., the DNA whose occurrence is to be detected and/or quantitated) and other DNA sequences. The amplified fetal DNA of interest and other DNA sequences are separated, using known techniques. Subsequent analysis of amplified DNA can be carried out using known techniques, such as: digestion with restriction endonuclease, ultraviolet light visualization of ethidium bromide stained agarose gels, DNA sequencing, or hybridization with allele specific oligonucleotide probes (Saiki, R.K., et al., Am. J. Hum. Genet., 43 (Suppl):A35 (1988)). Such analysis will determine whether polymorphic differences exist between the amplified "maternal" and "fetal" samples. In one embodiment, the amplification mixture is separated on the basis of size and the resulting size-separated fetal DNA is contacted with an appropriate selected DNA probe or probes (DNA sufficiently complementary to the fetal DNA of interest that it hybridizes to the fetal DNA of interest under the conditions used). Generally, the DNA probes are labelled (e.g., with a radioactive material, a fluorophore or other detectable material). After the size-separated fetal DNA and the selected DNA probes have been maintained for sufficient time under appropriate conditions for hybridization of complementary DNA sequences to occur, resulting in production of fetal DNA/DNA probe complexes, detection of the complexes is carried out using known methods. For example, if the probe is labelled, fetal DNA/labelled DNA probe complex is detected and/or quantitated (e.g., by autoradiography, detection of the fluorescent label). The quantity of labelled complex (and, thus, of fetal DNA) can be determined by comparison with a standard curve (i.e., a predetermined relationship between quantity of label detected and a given reading).

The present method has been used to identify Y-specific DNA in nucleated erythrocytes obtained from peripheral blood of pregnant women. This is described in Example 3. Briefly, candidate fetal cells from blood samples obtained from 19 pregnant women were isolated by flow sorting. The DNA in these cells was amplified for a 222 base pair (bp) sequence present on the short arm of the Y chromosome as proof that the cells were derived from the fetus. The amplified DNA was compared with standardized DNA concentrations; 0.1 to 1 ng fetal DNA was obtained in the 20 ml maternal samples. In 7/19 cases, a 222 bp band of amplified DNA was detected, consistent with the presence of male DNA in the isolated cells; 6/7 of these were confirmed as male pregnancies by karyotyping amniocytes. In the case of the female fetus, DNA prepared from cord blood at delivery also showed the presence of the Y chromosomal sequence. In 10/12 cases where the 222 bp band was absent, the fetuses were female. Thus, the Y chromosomal sequence was successfully detected in 75% of the male-bearing pregnancies, demonstrating for the first time that it is possible to isolate fetal gene sequences from maternal blood.

As described in Example 6, male (Y-specific) DNA has been detected in cells sorted from pregnant women at various points in gestation. Briefly, the mononuclear cell layer was isolated from venous blood samples obtained from women between 11 and 16 weeks gestation. Separation was carried out using Ficoll/Hypaque density centrifugation, followed by incubation with monoclonal antibodes (Anti-TfR, anti-Leu 4 and anti-Leu^{M}3) conjugated with a fluorescent marker or compound (fluorescein, phycoerythrin) and dual color analysis and flow sorting on a fluorescence-activated cell sorter. The cells that displayed green fluorescence, but not red fluorescence (TfR positive, Leu 4 negative, Leu M3 negative), were fetal nucleated cells and were separated from the remainder of the sample. These cells were lysed, after which the DNA was amplified and probed for the presence of a 397 bp sequence of the Y chromosome.

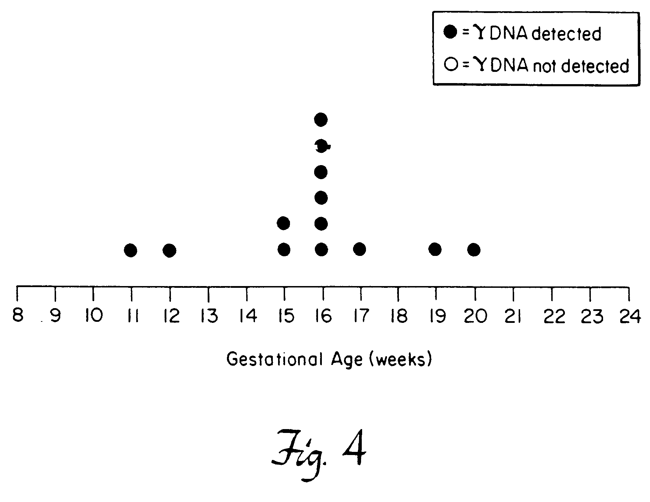

The results presented in Example 6 indicate the procedure allows the detection of the 397 bp sequence present in as little as 5 pg of male DNA. In addition, they suggest that there is a relationship between gestational age and detection of male DNA, as illustrated in Figure 4. This data suggests there may be a biologic "window" for transfer of fetal nucleated erithrocytes into maternal circulation.

The present method also has been used to distinguish female fetal DNA from maternal DNA. The two types of female DNA were distinguished using amplification of paternal polymorphism, as described in detail in Example 7. Briefly, venous blood samples were collected from women with uncomplicated pregnancies. Separation of fetal nucleated cells was conducted using Ficoll/Hypaque density centrifugation, followed by incubation with monoclonal antibodies (anti-TfR, anti-Leu 4 and anti-Leu M3) conjugated with a fluorescent marker (fluorescein, phycoerythrin) and dual color analysis and flow sorting on a fluorescence-activated cell sorter. Fetal nucleated cells identified by displaying green fluorescence (TfR positive), but not red fluorescence (Leu-4, Leu-3 negative), were collected and lysed. The DNA from the cells was amplified and probed for paternal sequences of the highly polymorphic region of chromosome 17, which allows the distinction of female fetal DNA from maternal DNA.

The results demonstrated that DNA sequences from the father can be identified in the autosomal chromosomes of the fetus. Consequently, the method of the present invention can be used to separate female fetal nucleated cells, as well as male fetal nucleated cells, from maternal blood. Thus, the method can be used for all DNA-based diagnostic procedures currently being used in other methods, such as amniocentisis.

Further support for of the present method's capability to identify Y-specific DNA in nucleated erthyroctyes obtained from peripheral blood of pregnant women is given by reconstruction experiments. As described in Example 8, male cord blood was added to blood obtained from non-pregnent females to simulate the presence of fetal cells in maternal blood. Briefly, venous blood samples were collected from healthy, non-pregnant women and the mononuclear cell layers isolated by Ficoll/Hypaque density centrifugation. Mononuclear cells from the umbilical cords of male infants (ranging from 10² to 10⁶ cells) were added to the mononuclear cell layers of the blood of non-pregnant women. The cord blood contains a large percentage of nucleated erythrocytes. The results obtained from these experiments were substantially similar to those obtained from pregnant women at various stages in gestation. Amplified sequences from the Y chromosome, consistent with the presence of male DNA, were detected when 10² male cells were added to the female cells.

The results of the work described above and in the Examples demonstrate that nucleated fetal cells have been isolated from maternal blood; genomic DNA has been extracted from the fetal cells and identified as being of fetal origin; fetal genes have been amplified using PCR; and selected DNA sequences have been identified in the fetal DNA. They demonstrate that for the first time, fetal DNA has been detected in cells isolated from maternal blood.

### Uses of the Present Method of Fetal Nucleated Cell Isolation and Fetal DNA Characterization

Thus, it has been demonstrated that fetal DNA can be obtained from fetal nucleated cells present in a maternal blood sample. The method of detecting and/or quantitating fetal DNA which is represented in Figure 1 is useful as a tool for prenatal assessment (e.g., as a means for assessing chromosomal abnormalities, for determining whether DNA associated with a disease is present, or for detecting Y-specific DNA). It is particularly useful because it is noninvasive and requires only a small sample of blood.

Fetal DNA sequences in fetal nucleated erythrocytes, isolated as described herein or by other means by which fetal nucleated cells can be separated from a maternal blood sample, can be analyzed or assessed for the occurrence of a DNA sequence or DNA sequences (gene(s) or gene portion(s)) which are of interest for diagnostic or other purposes. The DNA sequence(s) or gene(s)/gene portion(s) present in fetal cells are referred to herein as fetal DNA of interest. For example, the selected DNA whose presence or absence is to be determined and whose quantity can also be determined is the gene for a disease, such as cystic fibrosis, where the causative gene or gene portion has been cloned and sequenced; alternatively, it is a probe for X- or Y- specific DNA. The same procedure can also be used, with appropriate modifications (e.g., an appropriate DNA probe, time, temperature), to detect other genes or gene portions.

As used in a diagnostic context, such as to detect the gene known to cause cystic fibrosis, the present method is carried out as follows: Initially, a maternal blood sample (typically 20 ml.) is obtained and separated into component layers based on relative weights (e.g., by Ficoll-Hypaque density gradient centrifugation) to remove non-nucleated erythrocytes and produce a mononuclear cell layer. This results in production of a maternal blood sample enriched in fetal nucleated erythrocytes. The mononuclear cell layer is stained with at least one appropriate monoclonal antibody (e.g., one which is specific for the type of fetal nucleated cell to be separated from the sample). For example, a monoclonal antibody specific for fetal nucleated cells, such as anti-TfR antibody, described above, can be used. In general, the monoclonal antibody used bears a detectable label. Alternatively, a combination of selected labelled monoclonal antibodies, such as monoclonal antibodies specific for fetal nucleated cells (e.g., anti-TfR antibody) and monoclonal antibodies specific for maternal leucocytes (HLe-1 or L4 and M3), each labelled with a different fluorescent compound, can be used to remove essentially all maternal cells. Labelled cells are subsequently separated from one another using a known method, such as flow cytometry. Binding of the monoclonal antibodies to cells for which they are specific results in production of labelled monoclonal antibody-cell complexes. For example, in the case in which anti-TfR antibodies and HLe-1 are used, fetal nucleated erythrocytes are bound by anti-TfR antibody, to produce fetal nucleated erythrocytes/anti-TfR antibody complexes, and maternal leucocytes are bound by HLe-1 antibodies, to produce maternal leucocyte/HLe-1 antibody complexes. The fetal nucleated erythrocyte/anti-TfR antibody complexes are separated from maternal cell/HLe-1 antibody complexes, using, for example, flow cytometry. The fetal cells are lysed, to produce crudely extracted fetal DNA which is subsequently amplified, using, for example, PCR. This results in production of amplified fetal DNA, which is subsequently separated on the basis of size. Size-separated fetal DNA is contacted with labelled DNA probes (i.e., in prenatal detection of cystic fibrosis, a labelled DNA probe complementary to the gene associated with cystic fibrosis). If the fetal DNA contains DNA of interest (in this case, the gene associated with cystic fibrosis), fetal DNA of interest/labelled probe complexes are formed.

Fetal DNA of interest/labelled probe complexes are subsequently detected, using a known technique, such as autoradiography. Simple presence or absence of labelled fetal DNA of interest can be determined or the quantity of fetal DNA of interest present can be determined. In either case, the result is assessment of fetal DNA obtained from a maternal blood sample for selected DNA.

The occurrence of fetal DNA associated with diseases or conditions other than cystic fibrosis can also be detected and/or quantitated by the present method. In each case, an appropriate probe is used to detect the sequence of interest. For example, sequences from probes St14 (Oberle, I., et al., New Engl. J. Med., 312:682-686 (1985)), 49a (Guerin, P., et al., Nucleic Acids Res., 16:7759 (1988)), KM-19 (Gasparini, P., et al., Prenat. Diagnosis, 9:349-355 (1989)), or the deletion-prone exons for the Duchenne muscular dystrophy (DMD) gene (Chamberlain, J.S., et al., Nucleic Acids Res., 16:11141-11156 (1988)) are used as probes. St14 is a highly polymorphic sequence isolated from the long arm of the X chromosome that has potential usefulness in distinguishing female DNA from maternal DNA. It maps near the gene for Factor VIII:C and, thus, may also be utilized for prenatal diagnosis of Hemophilia A. Primers corresponding to sequences flanking the six most commonly deleted exons in the DMD gene, which have been successfully used to diagnose DMD by PCR, can also be used (Chamberlain, J.S., et al., Nucleic Acids Res., 16:11141-11156 (1988)). Other conditions which can be diagnosed by the present method include β-thalassemia (Cai, S-P., et al., Blood, 73:372-374 (1989); Cai, S-P., et al., Am. J. Hum. Genet., 45:112-114 (1989); Saiki, R.K., et al., New Engl. J. Med., 319:537-541 (1988)), sickle cell anemia (Saiki, R.K., et al., New Engl. J. Med., 319:537-541 (1988)), phenylketonuria (DiLella, A.G., et al., Lancet, 1:497-499 (1988)) and Gaucher disease (Theophilus, B., et al., Am. J. Hum. Genet., 45:212-215 (1989)). An appropriate probe (or probes) is available for use in the present method for assessing each condition.

It is also possible to separate fetal cells from maternal cells by means other than flow cytometry, as mentioned previously, and to analyze fetal nucleated erythrocyte DNA obtained in this way. Such separation procedures may be used in conjunction with or independent of flow cytometry. This is advantageous because lack of access to a flow cytometer, as well as expense, could limit potential applications of this technique. Thus, other methods of fetal cell separation can be used. The separation method used can result in elimination of unwanted cells ("negative selection") or isolation of rare but desirable cells ("positive selection").

For example, separation by immunomagnetic beads or by cell panning can be used. In this embodiment, the mononuclear cell layer is isolated, as described previously. This layer is then mixed with antibody-coated polymer particles containing magnetic cores (e.g., "Dynabeads"). These immunomagnetic beads are available coated with a variety of antibodies. For example, immunomagnetic beads coated with antibody to leucocyte antigens and antibody to mouse immunoglobulins, which can be subsequently conjugated to mouse monoclonal antibody against the human transferrin receptor, can be used. After mixing, the rosetted cells are isolated with a magnetic particle concentrator. In one embodiment, two sets of antibody-coated immunomagnetic beads are used in succession. First, the maternal leucocytes are depleted and then the remaining TfR positive cells are collected. Subsequent steps in the method (amplification, separation, contact with an appropriate DNA probe or probe set) are as described for cells separated by flow cytometry.

Mueller et al. (Lancet, 336: 197-200 (1990)) have described a method of isolating placenta-derived trophoblast cells in the blood of pregnant women using magnetic beads. This method included mixing 1 ml of monoclonal antibody hybridoma culture supernatant with 2 x 10⁷ magnetic beads precoated with sheep antibody to mouse IgG (Fc fragment) (Dynabeads M-450, Dynal AS, Oslo, Norway) and incubated overnight at room temperature. The coated beads were stored at 4°C and washed three times in ice-cold RPMI 1640 medium containing lithium heparin (10 IU/ml). The blood from the pregnant women was collected into tubes containing 10 IU of lithium per ml of whole blood, diluted 1:10 with RPMI containing lithium, and incubated with the antibody coated beads at 4°C overnight. The desired cells were bound to the antibody on the bead; the beads collected by means of a cobalt-samarium magnet. Although in this case the antibody was directed against trophoblast antigens, a similar technique can be utilized with, for example, antibody to cell surface antigens present on fetal nucleated erythrocytes and not present on maternal cells. An advantage to this particular technique is that an initial step which results in mononuclear cell isolation is not added. Additionally, the magnetic beads can be used for both positive (fetal cells) and negative (maternal cells) selection.

An alternative method of isolation can be a modification of the method described by R.J. Berenson et al. (J. of Immunol. Methods, 91: 11-19 (1986)) in which the high affinity between the protein avidin and the vitamin biotin was exploited to create an indirect immunoadsorptive procedure. In this technique, avidin was linked to cyanogen bromide activated sepharose 6MB beads and washed in an alternating fashion with coupling buffer (0.1 M NaHCO₃ in 0.5 M NaCl at pH 8.3) and washing buffer (0.1 M sodium acetate in 0.5 M NaCl at pH 4.5) and stored at 4°C. The blood cells were incubated with 1) murine monoclonal antibody, and 2) biotinylated goat anti-mouse immunoglobulin. A 3 ml column of gel was packed in a Pharmacia K 19/15 column. The treated cells were passed through the column in phosphate buffered saline containing 2% bovine serum albumin. Adherent cells were dislodged by mechanical agitation. This technique can be applied to fetal cell separation if the antibodies used recognize fetal cell surface antigens or maternal cell surface antigens, but not both. Variations in methods for conjugating antibodies to beads exist; examples include those described by Thomas and co-workers (Thomas, T.E., et al. (J. of Immuno. Methods, 120: 221-131 (1989)) and by deKretser and co-workers (deKretser, T.A., et al. (Tissue Antigens, 16: 317-325 (1980)). The use of an antibody-bound column does not require the preliminary isolation of the mononuclear cell fraction from whole blood.

Once the fetal cells are isolated from maternal blood, they may be cultured to increase the numbers of cells available for diagnosis, if desired. E. Fibach et al. (Blood, 73: 100-103 (1989)) have described a method that supports the growth of human hematopoietic progenitor cells. This step-wise method involves 1) initial culture in the presence of conditioned medium from human bladder carcinoma cells, 2) removal of leucocytes by harvest of non-adherent cells and lysis with monoclonal antibodies, and 3) reculture of cells in medium supplemented by recombinant erythropoietin.

Other methods of separating fetal nucleated cells from maternal cells can also be used, provided that they make it possible to differentiate between fetal cells and maternal cells, and to isolate one from the other.

A kit for use in carrying out the present method of isolating and detecting fetal DNA of interest, such as a chromosomal abnormality associated with a disease or other condition, in a maternal blood sample can be produced. It includes, for example, a container for holding the reagents needed; the reagents and, optionally, a solid support for use in separating fetal nucleated cell/specific antibody complexes from other sample components or for removing maternal cells complexed with specific antibody. For example, reagents in a kit to be used in detecting fetal DNA of interest after amplification of fetal DNA by PCR can include: 1) at least one antibody specific for a surface antigen characteristic of fetal nucleated cells but not specific for a surface antigen characteristic of maternal leucocytes; selected DNA primers for use in amplifying fetal DNA by PCR; and at least one DNA probe complementary to the fetal DNA to be detected (fetal DNA of interest). The kit, as indicated, can also include a solid support to be used in separating complexes formed from other samples components. Such solid support can be, for example, a glass slide, nitrocellulose filter, or immunomagnetic beads and can have affixed thereto an antibody selective for the antibody present in the fetal nucleated cell/specific antibody complexes.

The present invention will now be illustrated by the following examples, which are not intended to be limiting in any way.

### EXAMPLE 1 Antibody selection for isolation and sorting of fetal nucleated erythrocytes (NRBCs)

### Removal of maternal leucocytes from maternal blood using human leucocyte antigen (HLe-1)

The technique of fetal NRBC isolation began with an initial Ficoll-Hypaque density gradient centrifugation to remove the tremendously high number of non-nucleated erythrocytes in maternal blood. Peripheral blood was centrifuged and separated into a supernatant layer containing platelets, a mononuclear cell layer, and an agglutinated pellet consisting of non-nucleated erythrocytes and granulocytes. The mononuclear cell layer consisted of lymphocytes, monocytes, possible trophoblasts, and, due to their increased size and density, NRBCs and some reticulocytes. While the Ficoll-Hypaque centrifugation represented an initial enrichment in the proportion of fetal NRBCs present in the maternal sample, flow cytometry and cell sorting was used to improve the purity of the isolated cell population.

The mononuclear cell layer from peripheral blood samples in 63 pregnant women, 15 nonpregnant adults, and 39 umbilical cords, was stained with FITC-HLe-1 for flow cytometric analysis. Umbilical cord samples were used as a substitute for whole fetal blood. Representative histograms displaying fluorescence versus low-angle light scatter (an approximation of cell size) for each of the three groups were generated. Histogram peaks were identified that corresponded to leucocytes, erythrocytes and platelets. In 9 pregnant women, 7 nonpregnant adults and 12 umbilical cord samples, fluorescent (HLe-1 positive) and non-fluorescent (HLe-1 negative) cell populations were sorted for detailed microscopy after Wright-Giemsa staining. While the HLe-1 positive populations were always composed of leucocytes independent of the sample source, the HLe-1 negative populations differed.

In cord blood, the HLe-1 negative cells were non-nucleated and nucleated erythrocytes with occasional platelets. In the pregnant women, there were platelets, non-nucleated erythrocytes, and a very rare NRBC. In non-pregnant adults, only platelets and debris were seen. Thus, cord blood, with its high percentage of NRBCs, was used as a reference to establish cell sorting parameters. Microscopy confirmed the specificity of the antibody-antigen binding and that the sorted HLe-1 negative cells were relatively free from leucocyte contamination. These sorting parameters were utilized to isolate potential fetal NRBC on 40 pregnancies.

### Enrichment of fetal NRBC in maternal blood using transferrin receptor antigen (TfR)

The transferrin receptor (Newman, R., et al., Trends Biochem. Sci. 1:397-399 (1982)) is a surface glycoprotein important in cellular iron transport. The TfR is present on activated lymphocytes (Trowbridge, I.S., et al., Proc. Natl. Acad. Sci. USA, 78:3039-3043 (1981)), certain tumor cells (Greaves, M., et al., Int. J. Immunopharmac., 3:283-300 (1981)), and trophoblast cells (Galbraith, G.M.P., et al., Blood, 55:240-242 (1980)). Erythroblasts express the TfR on their cell surfaces from the BFU-E stage until nuclear extrusion (Loken, M.R., et al., Blood, 69:255-263 (1987)). Thus, TfR is an excellent "candidate antigen" for enrichment of fetal NRBCs found in maternal blood. Monoclonal antibody against TfR is available as both a fluorescein conjugate (Becton-Dickinson catalog #7513) and a phycoerythrin (PR) conjugate (gift of Dr. Michael Loken, Becton-Dickinson). The mononuclear cell layer was isolated from peripheral blood samples in 6 pregnant women, 4 non-pregnant adults, and 3 newborn umbilical cords for TfR analysis and microscopy. Representative histograms of fluorescence versus light scatter from these three groups were generated.

Whereas umbilical cord samples had a large population of fluorescent (TfR positive cells) that were heterogenous in size, non-pregnant adults and pregnant adults had smaller percentages of fluorescent cells that clustered in discrete groups. In addition, there were slight differences in the percentages of TfR positive cells in the pregnant (mean = 0.83) versus non-pregnant (mean = 0.32) samples studies.

Microscope studies of the TfR positive cells were performed using Wright-Giemsa stain for morphology and Kleihauer-Betke technique for the detection of fetal hemoglobin (Kleihauer, E., et al., Kiln Wochenschr., 35:637-638 (1957)). In the umbilical cord samples, large numbers of nucleated and non-nucleated erythrocytes containing fetal hemoglobin and occasional leucocytes were identified visually. In the pregnant women, the predominant cell types were nucleated and non-nucleated erythrocytes containing fetal hemoglobin, although leucocytes were infrequently observed. In contrast, the samples from the non-pregnant controls consisted almost exclusively of lymphocytes and monocytes. Because trophoblast cells express TfR, it was postulated that they might be present in the sorted population from the pregnant women; none was detected.

### Dual antibody analysis

Because both antibodies enriched the proportion of NRBCs present, but did not completely exclude other cell types in the sorted samples, combinations of antibodies were used to isolate pure populations of fetal NRBCs. Preliminary dual antibody studies were performed using PE-conjugated TfR and FITC-conjugated HLe-1. NRBCs are TfR positive and HLe-1 negative, whereas maternal leucocytes are HLe-1 positive. These experiments worked well and resulted in separation of maternal leucocytes.

Thus, the work described above defined flow cytometric parameters for enrichment and sorting of NRBCs in peripheral blood from pregnant women. In addition, microscopic studies revealed that morphologic differences occur in mononuclear cell populations derived from venous blood samples in pregnant versus non-pregnant adults.

### EXAMPLE 2 DNA hybridization studies in HLe-1 negative cells sorted from maternal blood

To confirm fetal origin of the cells sorted as described in Example 1, Y chromosomal probes were used because it is the Y chromosome that is unquestionably fetal in origin. The assessments were designed to study whether the presence of Y chromosomal DNA in maternal blood as detected on autoradiographs performed antenatally correlated with the subsequent birth of a male infant.

### DNA isolation

HLe-1 negative cells from cord blood and pregnant women were sorted into test tubes. Conventional methods of DNA isolation as well as modification of cruder methods (Lau, Y-F., et al. Lancet, 1:14-16 (1984); McCabe, E.R.B., et al., Hum Genet., 75:213-216 (1987)) were attempted without success in detecting Y chromosome derived bands on Southern Blots. All were limited by the small numbers of cells present.

### EXAMPLE 3 Direct hybridization to cells deposited on filters

In order to circumvent technical problems associated with DNA isolation, a method of direct DNA hybridization to cells flow sorted onto nitrocellulose filters was developed (Bianchi, D.W., et al., cytometry, 8:197-202 (1987)). In control experiments, the sex of a newborn was determined from as few as 50 sorted cord blood leucocytes or 5,000 HLe-1 negative cells (a mixture of nucleated and non-nucleated cells).

The methodology was then applied to detection of Y chromosomal sequences in HLe-1 negative cells sorted from peripheral blood samples in 40 women between 8½ and 38 weeks gestation. Results were the following:

| Dot Blot Hybridization Y Chromosomal Probe | with Delivered Male Infant | Delivered Female Infant | Lost to Follow-up |
|---|---|---|---|
| + | 3 | 2 | 0 |
| - | 21 | 12 | 2 |

It was concluded that hybridization with this probe was not predictive of male pregnancy. The possibility exists that there was fetal DNA present on the filters where DNA hybridization occurred, but that this DNA bound to the Y probe nonspecifically. Thus, the filters interpreted as "positive" for male DNA might actually have been "positive" for fetomaternal hemorrhage.

### EXAMPLE 4 Use of the polymerase chain reaction (PCR) to amplify gene sequences in sorted fetal cells

PCR, which has a capacity for making 10⁶ Copies of rare target gene sequences, was used to amplify gene sequences in sorted fetal cells. Optimum conditions for PCR, given the minute amounts of DNA expected after a fetal cell sort (approximately 1 pg to 100 ng), were determined. Experimental conditions were modified as new information became available. For example, Taq polymerase was used instead of Klenow fragment of E. Coli DNA polymerase (Kogan, S.C. et al., New England J. Med. 317:990 (1987)) because of its increased specificity in DNA replication.

Initially, studies were performed on repeated sequences from the long arm of the Y chromosome, probe Y431-Hinfa (given by Dr. Kirby Smith, Johns Hopkins University, Baltimore, MD) and the short arm of the Y chromosome, probe Y411 (Given by Dr. Ulrich Muller, Children's Hospital, Boston, MA). Repeated sequences were selected because they would create a stronger amplification signal from a rare male fetal cell. Y411 is identical to Y156 (Muller, U., et al.,Nucleic Acids Res., 14:1325-1329 (1986)), is repeated 10-60 fold, and is absolutely Y specific on Southern blots. Sequence Y431 has autosomal homology in females that limited its usefulness in sex determination.

### PCR standardization

To define the minimum amount of DNA detectable in maternal blood, a series of standardization experiments were done. DNA from male and female individuals was prepared in tenfold dilutions (1 pg to 1 mcg) and amplified using the standard reagents in the GeneAmp kit (Perkin-Elmer Cetus cat #N801-0055) on a Perkin-Elmer DNA Thermal Cycler. Primers 411-01 and 411-03 were designed to amplify a 222 base pair (bp) sequence within probe Y411. The number of amplification cycles varied between 18 and 30. Amplified DNA samples were electrophoresed on agarose gels, transferred to nylon filters, and hybridized to ³²P-labeled Y411 probe. While it appeared possible to detect Y specific bands on autoradiographs in lanes containing as little as 10 pg of male DNA, results were often muddled by the presence of amplified DNA in female lanes or control lanes containing no added DNA. The phenomenon of "false positive amplification" has now received universal recognition (Lo, Y-M.D., et al., Lancet, 2:697 (1988); Kwok, S., et al., Nature, 339:237-238 (1989)).

### Elimination of "false positive" amplification

Due to the limited amount of starting material in a fetal cell sort, every effort was made to eliminate background amplification in order to determine which fetuses truly possess Y chromosomal DNA. Thus, measures were taken to prevent aerosol contamination of male DNA. All PCRs were performed under sterile conditions, wearing gloves, and using positive displacement pipettes. All reagents were prepared in a sterile manner and incubated overnight prior to PCR with a restriction endonuclease having a digestion site within the target sequence. These precautions resulted in a significant decrease and virtual absence of false positive amplification, as monitored by running control reactions with all reagents but no DNA.

### Successful isolation and amplification of fetal gene sequences from NRBCs in maternal blood

After eliminating sources of DNA contamination and determining that as little as 10 pg of male DNA (1 cell = 7 pg of DNA) could be detected after PCR amplification, candidate fetal cells from the peripheral blood of 19 women at 12½ to 17 weeks gestation were sorted. Monoclonal antibody against TfR was used to identify the presumed NRBC. The DNA in the sorted cells was amplified for the 222 bp sequence in probe Y411 as proof that the cells were derived from the fetus in male pregnancies. In 7/19 cases the 222 bp band of amplified DNA was detected on autoradiographs, consistent with the presence of male DNA in the isolated cells; 6/7 of these were confirmed as male pregnancies by karyotyping amniocytes. In the case of one female fetus, repeat studies at 32 weeks gestation and cord blood at delivery also showed the presence of the Y chromosomal sequence. This result might be explained by a low level of sex chromosome mosaicism, XX/XY chimerism (Farber, C.M., et al., Hum. Genet., 82:197-198 (1989)), or the presence of the Y411 sequence in single copy on the X chromosome or autosomes. In 10/12 cases where the 222 bp was absent, the fetuses were female. Therefore, detection of the Y chromosomal sequence was successful in 6/8 or 75% of the male-bearing pregnancies. In the two pregnancies where male DNA was not detected, there may have been fetomaternal blood group incompatibility. Alternatively, there may not have been fetomaternal hemorrhage or the number of NRBCs present may have been below the limit of sensitivity for detection of DNA. The conditions used made it possible to detect a minimum of 100 pg of fetal DNA, or the equivalent of 15 fetal cells. The limit of sensitivity can be improved by extending the number of cycles used in PCR. This work demonstrated that for the first time, fetal DNA was detected in cells isolated from maternal blood.

To further decrease false positive amplification and permit detection of fetal DNA at the single cell level on agarose gels, PCR is being carried out using primers derived from a single copy of sequence specific for the long arm of the Y chromosome, pY49a (Guerin, P., et al.,Nucleic Acids Res., 16:7759 (1988)). In preliminary experiments using 6O cycles of PCR, Y chromosomal DNA is visible on ethidiumbromide stained agarose gels. This extraordinary degree of sensitivity will now be applied to DNA from sorted fetal cells.

### EXAMPLE 5 Determination of the Volume, Morphology and Universality of Fetomaternal Hemorrhage

### a. General Strategy

It is also possible, because of the availability of the present method of isolating fetal nucleated cells from blood obtained from a pregnant woman, to determine whether fetal cells can be found in the maternal blood in all pregnancies. A data base can be created that can provide information on the number and type of fetal cells circulating in maternal blood as pregnancy progresses. Based on previous work. it is anticipated that there will be a normal range of values that is dependent on gestational age; deviation from these values will be studied as a potential indication of a pregnancy at risk. Specifically, large amounts of fetal blood in the maternal circulation may be correlated with placental abnormalities, threatened miscarriage and intrauterine growth retardation.

Maternal venous blood samples are collected from pregnant women, generally prior to any invasive procedures. In general, a single 20 ml. venous blood sample will be obtained. In a subgroup of patients, permission will be sought to draw blood samples every 4 weeks to follow changes in numbers of fetal cells present. Blood is collected in EDTA, diluted 1:1 with Hanks Balanced Salt Solution (HBSS), layered over a Ficoll-Hypaque column (Pharmacia) and spun at 1400 rpm for 40 minutes at room temperature. The mononuclear cell layer will be isolated, washed twice with HBSS, and stained with fluorescent monoclonal antibodies. For example, this can be a combination of fluorescein isothiocyanate-conjugated antitransferrin receptor (TfR) and phycoerythrin-conjugated anti-monocyte antibodies (M3, Becton-Dickinson catalog #7497) and anti-lymphocyte antibodies (L4, Becton-Dickinson catalog #7347). The staining occurs on ice, in phosphate buffered saline (PBS) containing 2% fetal calf serum and 0.1% sodium azide. The cells are washed in PBS prior to flow cytometry. Analysis and sorting are performed on a Becton-Dickinson FACS-IV interfaced with a Consort 40 program. Data will be acquired on the relative size and fluorescence (in two colors) of the analyzed cells. Cells that are fluorescent in the green wavelength (TfR positive) and not fluorescent in the red wavelength (L4 and M3 negative) will contain the presumed fetal NRBCs. The percentage of these cells in the mononuclear cell layer arerecorded and analyzed as a function of gestational age. These cells are sorted for microscopy and PCR amplification. In addition, cells that are not fluorescent in the green wavelength (TfR negative) but are fluorescent in the red wavelength (L4 and/or M3 positive) are sorted as a presumed maternal leucocyte population and source of maternal DNA polymorphisms.

An additional benefit of studying nucleated fetal cells in maternal blood is that the amount of fetal DNA present can be extrapolated to determine the extent of fetomaternal hemorrhage in normal and unusual pregnancies. In the pregnancies studied, an average amount of 1 ng of fetal DNA (corresponding to 150 NBCRs) was present. Using published values of the number of NRBCs per liter of fetal blood at 16 weeks (3.6 x 10⁹) (Millar, D.S., et al., Prenat. Diagnosis, 5:367-373 (1985); (Forestier, F., et al., Pediatr. Res., 20:342-346 (1986)) and doing simple algebra, these results were calculated to be consistent with 2-20 µl hemorrhage of fetal blood into maternal circulation. This is a trivial amount when compared with the fetoplacental blood volume at 16 weeks, about 20 ml. It is important to validate and extend these results to generate normative data regarding fetomaternal transfusion in early pregnancies. It will be equally important to correlate deviations from the expected results with pregnancy complications.

### Example 6 Detection of Male DNA in Cells Sorted from Pregnant Women at Different Points in Gestation

Venous blood samples (20 ml) were collected in EDTA from healthy women with uncomplicated pregnancies, prior to invasive diagnostic procedures, at different points in gestation. The mononuclear cell layer was isolated by Ficoll/Hypaque density centrifugation and incubated with the monoclonal antibodies fluorescein (FITC)-conjugated anti-TfR, phycoerythrin (PE)-conjugated anti-Leu 4 and PE-conjugated anti-Leu M3 (Becton-Dickinson). Dual color analysis and flow sorting were performed on a fluorescence-activated cell sorter.

Cells that display green fluorescence but not red fluorescence (TfR positive, Leu 4 negative, Leu M3 negative) were collected into sterile micro test tubes and frozen at -20°C. Prior to polymerase chain reaction amplification, the cells were lysed by boiling. The polymerase chain reaction (PCR) was performed under standard conditions using standard reagents as described in Example 4. The primers used to amplify material from the Y chromosome define a 397 base pair (bp) sequence. After PCR, the patient samples were analyzed with conventional Southern blots using ³²P labelled probe. Ethidium bromide stained agarose gels and autoradiographs were examined for the presence of the 397 bp band, which is considered significant only if reagent controls do not reveal false positive amplification.

Under the reaction conditions described above, it was possible to detect the 397 bp male specific band if 5 pg of male DNA was present. This is approximately the amount of DNA present in one cell. When excess female DNA (500 ng) was added to the reaction mixture, the male specific band was consistently detectable at 100 pg.

Figure 4 represents a summation of samples obtained from twelve women bearing male fetuses. These samples were taken at different times in pregnancy, and one woman was sampled twice. The data indicates that there is a relationship between gestational age and the detection of male DNA. This implies a potential biologic "window" for the transfer of fetal nucleated erythrocytes into the maternal circulation.

### Example 7 Detection of Female Fetal DNA by Amplification of Paternal Polymorphisms

Venous blood samples (20 ml) were collected in EDTA from healthy women with uncomplicated pregnancies. The mononuclear cell layer was isolated by Ficoll/Hypaque density centrifugation and incubated with the monoclonal antibodies fluorescein (FITC)-conjugated anti-TfR, phycoerythrin (PE)-conjugated anti-Leu 4 and PE-conjugated anti-Leu M3 (Becton-Dickinson). Dual color analysis and flow sorting were performed on a fluorescence-activated cell sorter.

Cells that display green fluorescence but not red fluorescence (TfR positive, Leu 4 negative, Leu M3 negative) were collected into sterile micro test tubes and frozen at -20°C. Additionally, cells that displayed red fluorescence but not green fluorescence (TfR negative, Leu 4 positive, Leu M3 positive) were collected in an identical manner. Prior to polymerase chain reaction (PCR) amplification, the cells were lysed by boiling. PCR was performed using buffers containing 1 mM MgCl₂. The primers used in PCR amplify a highly polymorphic region of chromosome 17. Amplified DNA sequences correspond to blocks of genes transmitted directly from parent to child. As a result of the high degree of individual variation in these sequences, it is uncommon for two parents to manifest identical DNA patterns. Thus, it is possible to demonstrate inheritance of the paternal sequences in the sorted fetal cells. Since these sequences are from chromosome 17, they are independent of fetal sex, and may be used to distinguish female fetal DNA from maternal DNA. Amplified DNA was separated by electrophoresis through ethidium bromide stained agarose gels. The DNA was transferred to nylon filters and probed using ³²P labeled sequence. The maternal DNA, paternal DNA, TfR⁺ cells, and TfR⁻ cells were then compared.

In 5 of 10 pregnant women, it was possible to show the presence of paternal sequences in the sorted candidate fetal cell population. In the other 5 women, no differences were seen between the maternal DNA and the DNA obtained from the candidate fetal cells.

### Example 8 Reconstruction Experiments Using Non-Pregnant Female Blood and Added Male Cord Blood to Simulate the Presence of Fetal Cells in Maternal Blood

Venous blood samples (20 ml) were collected in EDTA from healthy non-pregnant women. Umbilical cord blood samples (10 ml) were collected in EDTA from normal newborns. The mononuclear cell layer was isolated by Ficoll/Hypaque density centrifugation. Cell counts were performed with a hemocytometer. Separate aliquots of cells were made containing: 1) female cells alone; 2) female cells plus 10² added male cord blood cells; 3) female cells plus 10³ added male cord blood cells; 4) female cells plus 10⁴ added male cord blood cells; 5) female cells plus 10⁵ added male cord blood cells; 6) female cells plus 10⁶ added male cord blood cells; 7) male cord blood cells alone. The separate aliquots were then incubated with the individual monoclonal antibodies being tested. Analysis and sorting were performed using a flow cytometer. For each aliquot, a bivariate histogram was obtained, and gating parameters were established for antibody positive and antibody negative cells. The sorted cells were collected into sterile micro test tubes and frozen at -20°C. PCR amplification was performed with primers that detect a 397 bp sequence unique to the Y chromosome. The presence of a band at 397 bp in autoradiographs was used to confirm the presence of male unbilical cord blood cells in sorted samples.

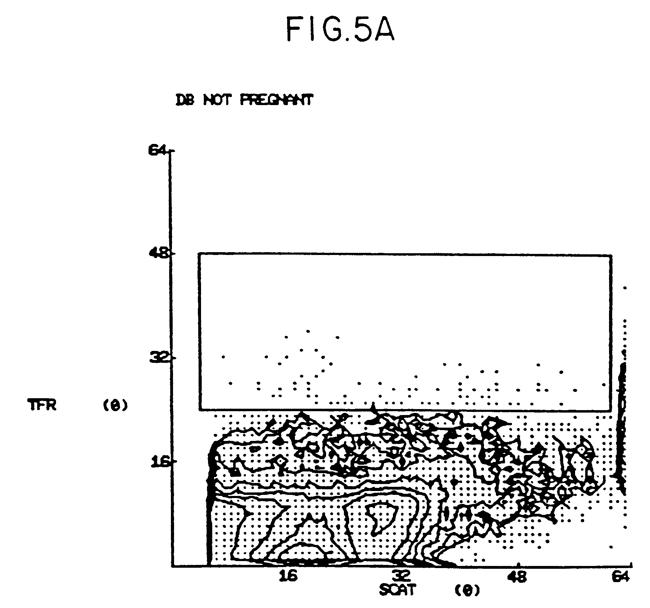

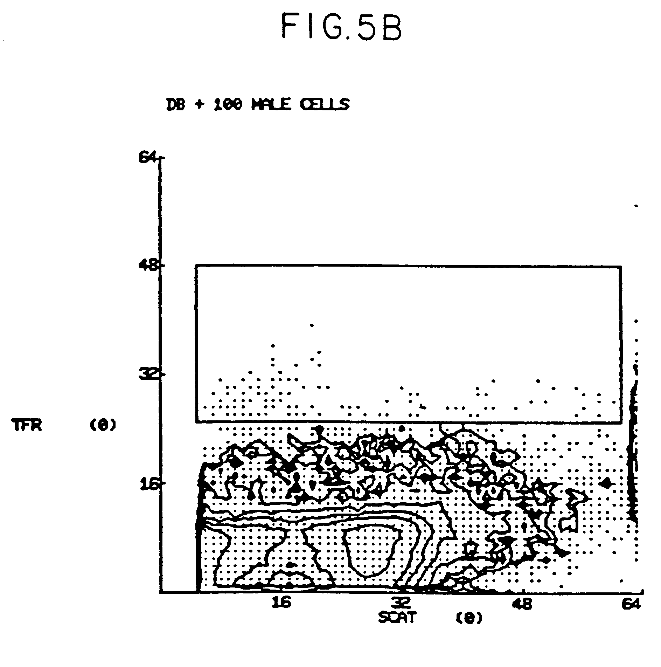

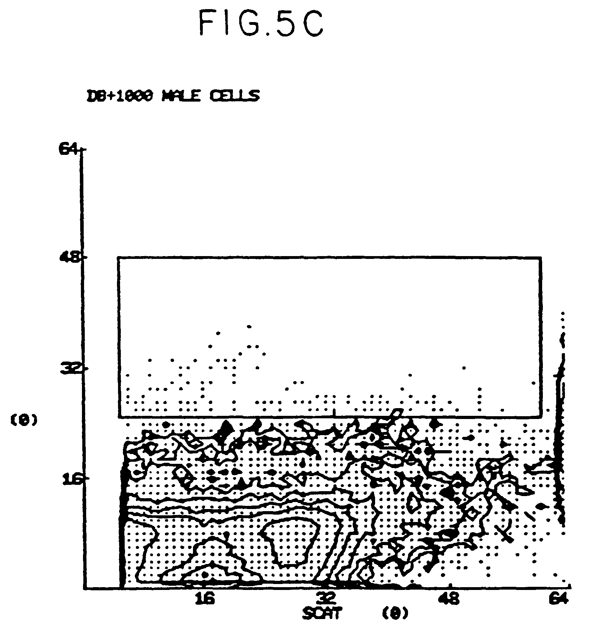

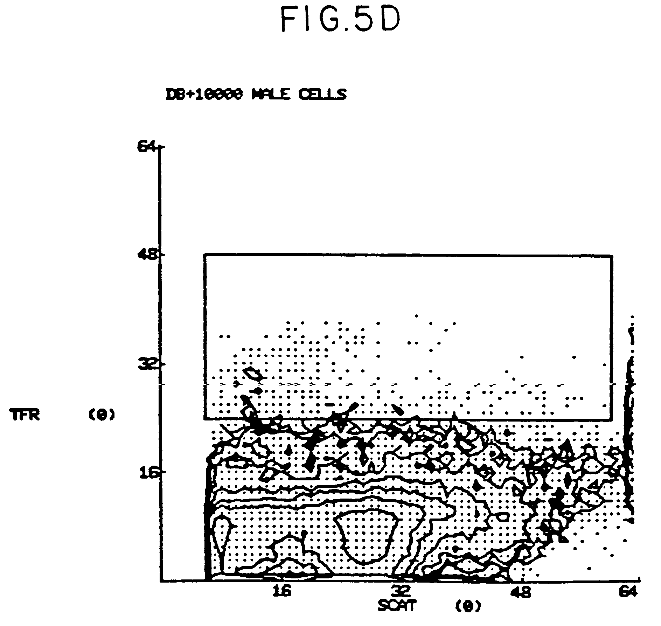

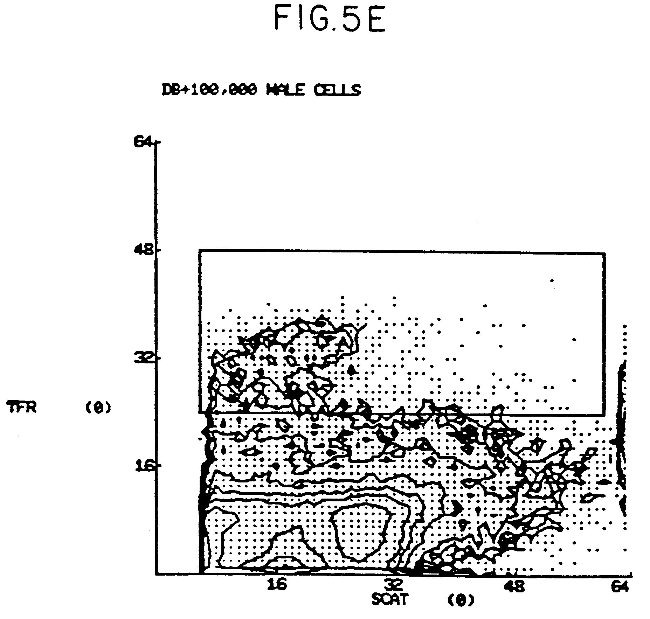

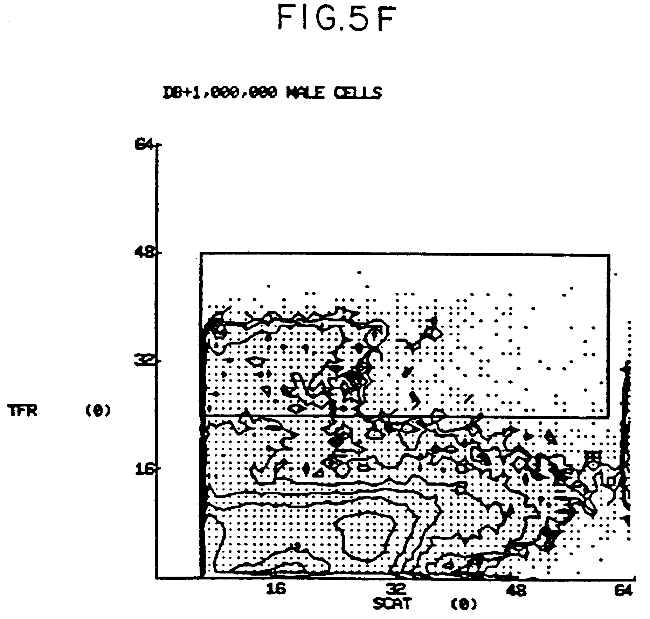

Figure 5 shows the histograms obtained when FITC-anti transferrin receptor is used. In the non-pregnant female, 0.1% of the mononuclear cells react with the antibody. In male cord blood, 24.9% of the mononuclear cells react with the antibody. With the addition of more and more umbilical cord cells to the non-pregnant female cells, an increased percentage of cells that react with the antibody is seen.

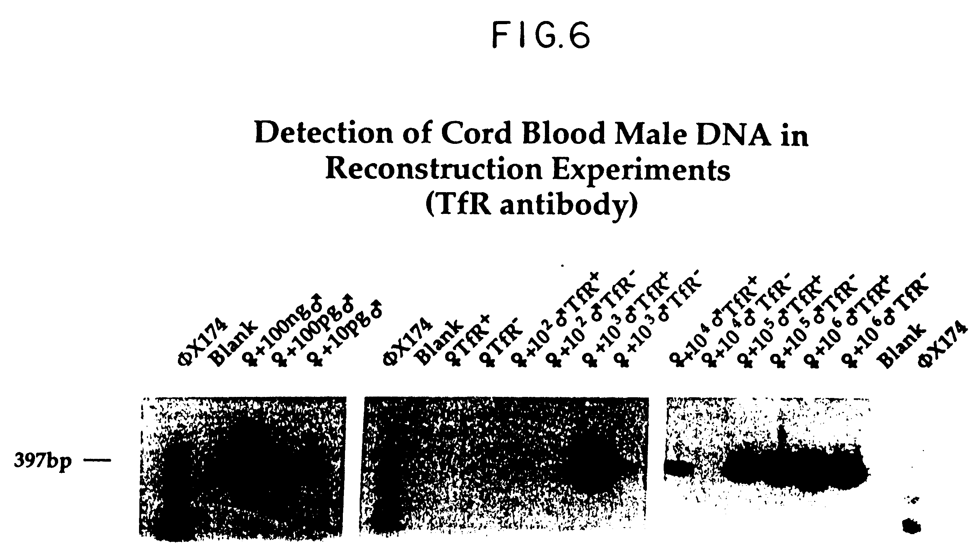

Figure 6 shows that male DNA is detected in the TfR⁺ cells when 10²-10⁶ male cells are added. Male DNA is detected in the TfR⁻ cells when 10⁵-10⁶ male cells are added. This results from the presence of male white blood cells in the TfR⁻ population.

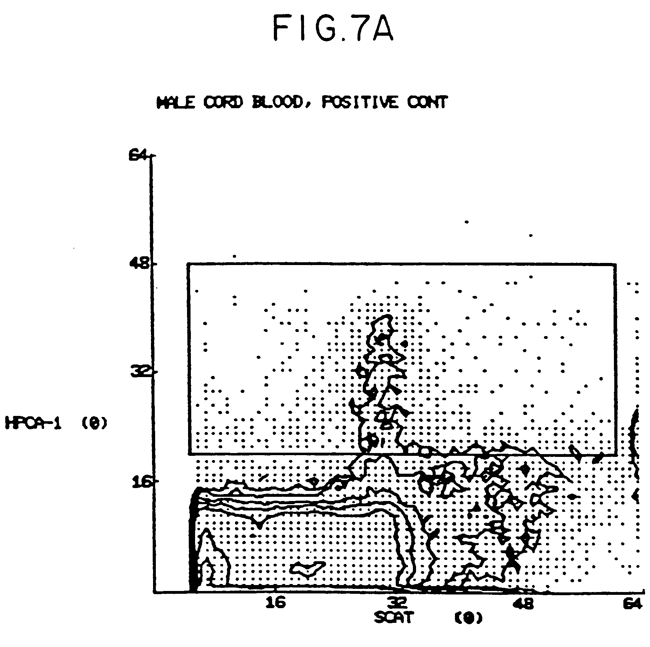

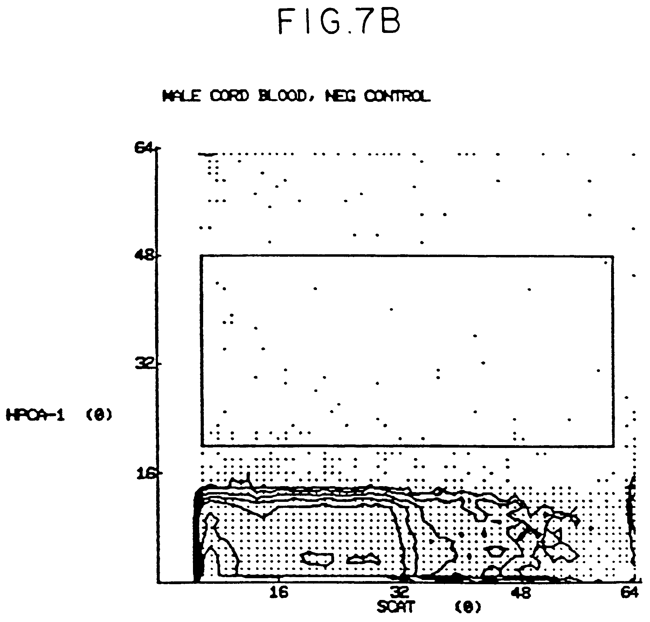

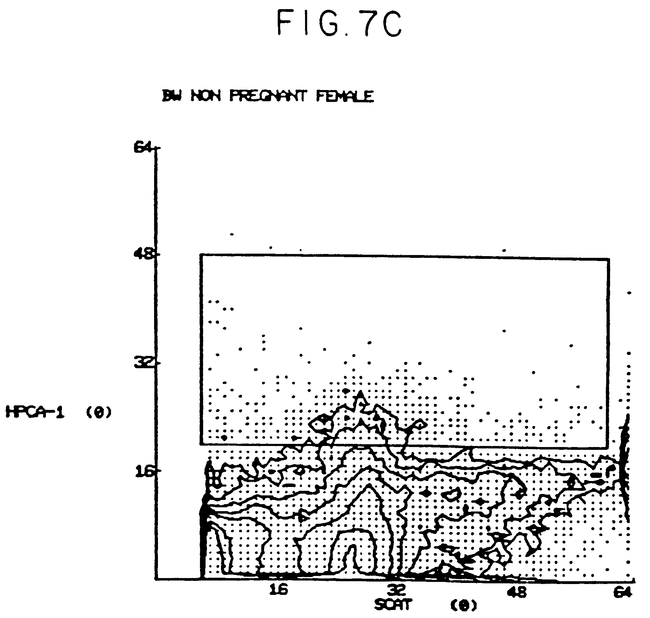

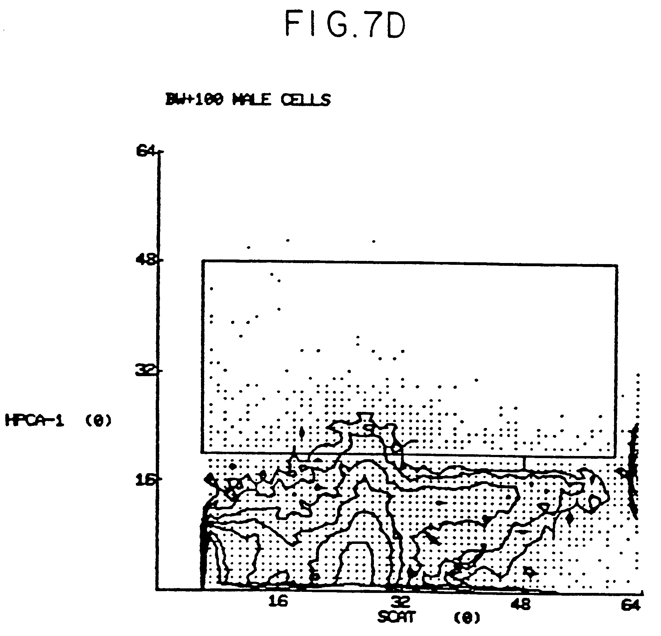

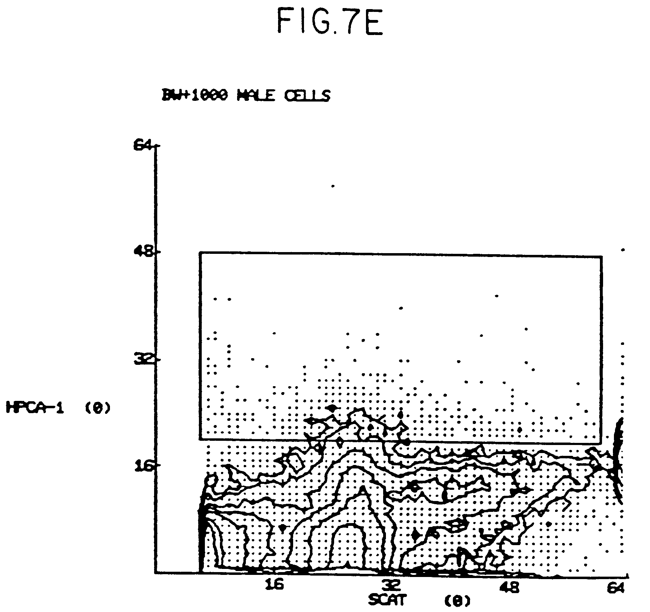

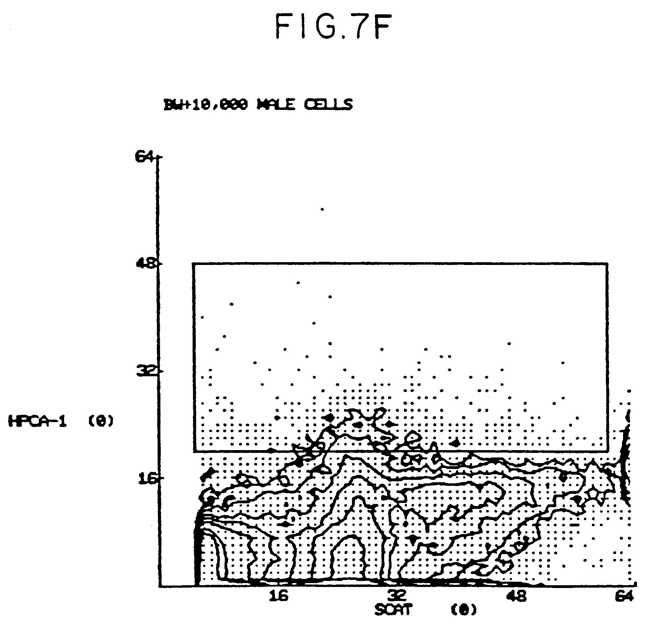

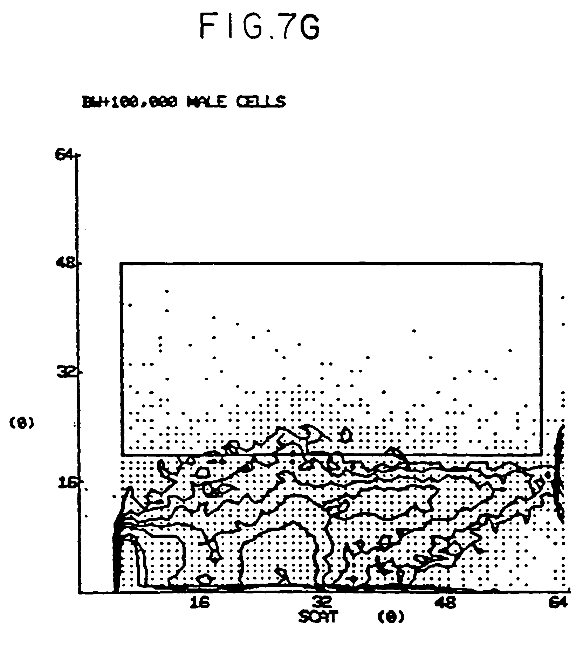

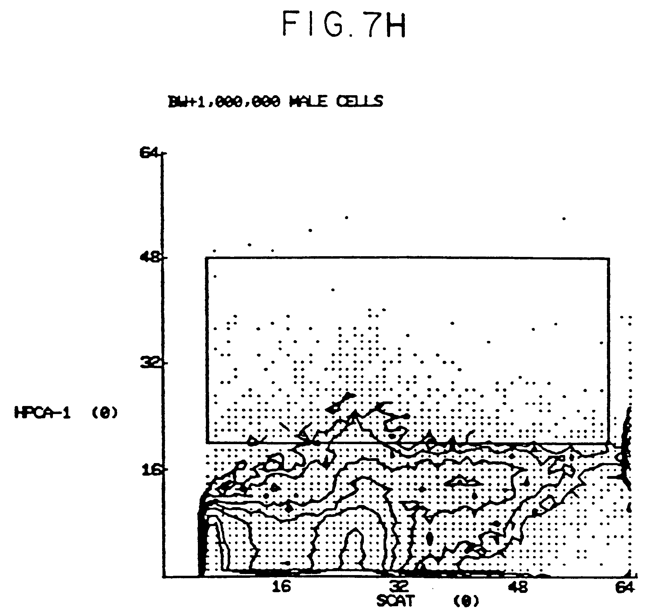

Figure 7 shows the histograms obtained when anti HPCA-1 antibody is used. In the non-pregnant female, 0.9% of the mononuclear cells react with antibody. In umbilical cord blood, a well-defined population of cells is seen, but the percentage is only 1.1%. Thus, the addition of umbilical cord blood cells to the non-pregnant female cells is not seen on the histograms as clearly as with the transferrin receptor antibody. An increased number of HPCA⁺ cells were collected as the amounts of added cord blood cells increased.

In agarose gels, the 397 bp band consistent with DNA was detected in the HPCA⁺ cells when 10³-10⁵ male cells were added to the female cells. Male DNA was detected in agarose gels in the HPCA cells when 10⁶ male cells were added to the female cells.

### Example 9 In situ Hybridization Using Molecular Probes Recognizing Individual Chromosomes in Flow Sorted Nucleated Erythrocytes

To demonstrate diagnostic utility of the present invention, a DNA probe set was constructed of chromosome specific probes that provided both good signal to noise ratios and good spatial resolution of the fluorescent signals. Accordingly, specific probes were developed for five chromosomes frequently seen as liveborn aneuploidies; chromosomes 13, 18, 21, X and Y. A probe for chromosome 1 was used as a control. In constructing the probes, the general strategy was to identify a starting clone that mapped to the desired chromosomal region by multiple genetic and physical methods, and then to use that clone to identify a matching cosmid "contig" which was then used as a hybridization probe.

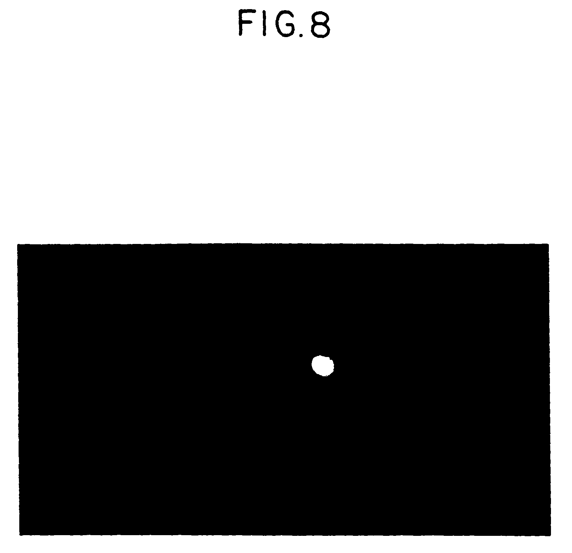

Hybridization of the high copy number repeat sequences was suppressed by inclusion of total genomic human DNA, and the chromosomal specificity verified by hybridization to metaphase spreads. The probes gave sharp, punctate fluorescent signals in interphase cells that was easily discriminated and enumerated. The Y probe used in this study was pDP97, a repetitive clone (a 5.3 kb EcoRI Y fragment from cosmid Y97 subcloned into EcoRI site of pUC-13). All probes were labeled with biotin, hybridized under suppression conditions, and specific hybridization detected by conjugated streptoavidin-FITC, which showed as a single "dot" in the FITC image. As illustrated in Figure 8, the Y chromosome was detected by in situ hybridization of the pDP97 probe for the Y chromosome in a fetal nucleated red blood cell. Thus, prenatal diagnosis for chromosomal abnormalities could be performed on fetal cells isolated from maternal blood.

## Claims

1. A method of separating fetal nucleated cells (e.g. fetal nucleated erythrocytes) present in a sample of blood obtained from a pregnant woman, comprising separating fetal nucleated cells present in the sample of blood from other cells present in the blood on the basis of an antigen present on fetal nucleated cells, or present on other cells present in the blood, but not present on both.

2. An in vitro method of separating fetal nucleated erythrocytes present in the blood of a pregnant woman, comprising sorting fetal nucleated erythrocytes present in the blood on the basis of an immunologic feature which is characteristic of fetal nucleated erythrocytes or characteristic of cells other than fetal nucleated erythrocytes, but not characteristic of both.

3. A method of separating fetal nucleated cells present in a sample of whole blood obtained from a pregnant woman, comprising contacting the sample of whole blood with at least one antibody which is specific for an antigen present on fetal nucleated cells, but not present on maternal cells, under conditions appropriate for binding of the anibody with the antigen present on fetal nucleated cells, thereby forming antibody-fetal nucleated cell complexes and separating antibody-fetal nucleated cell complexes from the sample.

4. A method of separating fetal nucleated erythrocytes present in a maternal blood sample, comprising the steps of:
a) Contacting the blood sample with 1) a first monoclonal antibody, which is specific for an antigen present on fetal nucleated erythrocytes but not for maternal leucocytes and 2) a second monoclonal antibody, which is specific for an antigen present on maternal leucocytes but not on fetal nucleated erythrocytes, under conditions appropriate for binding of monoclonal antibody to specific antigen, thereby producing fetal nucleated erythrocyte/first monoclonal antibody complexes and maternal leucocyte/second monoclonal antibody complexes; and
b) separating fetal nucleated erythrocyte/first monoclonal antibody complexes from maternal leucocyte/second monoclonal antibody complexes.

5. The method of Claim 4 wherein the first monoclonal antibody and the second monoclonal antibody are fluorescently labelled, each with a different fluorescent material and separation in step (b) is by flow cytometry.

6. A method of separating fetal nucleated erythrocytes present in a sample of blood obtained from a pregnant woman, comprising he steps of:
a) combining the maternal sample with at least one detectable monoclonal antibody selective for fetal nucleated erythrocytes, under conditions appropriate for binding of detectable monoclonal antibody with fetal nucleated erythrocytes, to produce bound detectable monoclonal antibody; and
b) separating bound detectable monoclonal antibody from the sample.

7. The method of Claim 6 further comprising,
a) in step (a), combining the maternal sample with at least two detectable monoclonal antibodies, wherein the first of said detectable monoclonal antibodies is selective for antigens present on mature human leucocytes and for antigens present on very immature erythrocyte precursors, but not for mature nucleated erythrocytes; the second of said detectable monoclonal antibodies is selective for an antigen which is present on the surface of fetal nucleated erythrocytes; the second of said detectable monoclonal antibodies is selective for an antigen which is present on the surface of fetal nucleated erythrocytes, but not for antigens present on mature human leucocytes or antigens present on very immature erythrocytes; and the two detectable monoclonal antibodies are separately detectable, to produce a first bound detectable monoclonal antibody and a second bound detectable monoclonal antibody; and
b) in step (b), separating the first bound detectable monoclonal antibody and the second bound detectable monoclonal antibody from one another; and for example wherein the first of said detectable monoclonal antibodies is selected from the group consisting of HLe-1, L4 and M3 HLe-1 and the second of said detectable monoclonal antibodies is anti-TfR antibody.

8. A method of separating fetal nucleated cells present in the blood of a pregnant woman, comprising the steps of:
a) separating a sample of blood obtained from the pregnant woman into constituent layers, said constituent layers including a mononuclear cell layer; and
b) separating fetal nucleated cells present in the mononuclear cell layer from other cells present in the mononuclear cell layer on the basis of an antigen present on fetal nucleated cells, present on other cells present in the mononuclear cell layer, but not on both.

9. The method of Claim 8 wherein the sample of blood obtained from the pregnant woman is separated into constituent layers in step (a) by density gradient centrifugation and the fetal nucleated cells are fetal nucleated erythrocytes.

10. A method of separating fetal nucleated erythrocytes present in the blood of a pregnant woman, comprising enriching the proportion of fetal nucleated erythrocytes present in a sample of peripheral blood obtained from the pregnant woman to produce a maternal sample enriched in fetal nucleated erythrocytes, and sorting fetal nucleated erthrocytes present in the maternal sample enriched in fetal nucleated erythrocytes on the basis of an immunologic feature which is characteristic of fetal nucleated erythrocytes or characteristic of cells other than fetal nucleated erythrocytes, but not characteristic of both.

11. The method of Claim 10 wherein the proportion of fetal nucleated erythrocytes present in the sample of peripheral blood is enriched by separating the sample by density gradient centrifugation into constituent layers and removing the mononuclear cell layer.

12. A method of separating fetal nucleated erythrocytes present in a maternal blood sample, comprising the steps of:
a) separating the maternal blood sample into constituent layers, said constituent layers including a mononuclear cell layer;
b) separating the mononuclear cell layer from the constituent layers produced in step (a);
c) contacting the mononuclear cell layer with 1) a first monoclonal antibody, which is specific for an antigen present on fetal nucleated erythrocytes but not for maternal leucocytes and 2) a second monoclonal antibody, which is specific for an antigen present on maternal leucocytes but not on fetal nucleated erythrocytes, under conditions appropriate for binding of monoclonal antibody to specific antigen, thereby producing fetal nucleated erythrocyte/first monoclonal antibody complexes and maternal leucocyte/second monoclonal antibody complexes; and
d) separating fetal nucleated erythrocyte/first monoclonal antibody complexes from maternal leucocyte/second monoclonal antibody complexes.

13. The method of Claim 12 wherein the first monoclonal antibody and the second monoclonal antibody are fluorescently labelled, each with a different fluorescent material and separation in step (d) is by flow cytometry.

14. A method of separating fetal nucleated erythrocytes present in a sample of blood obtained from a pregnant woman, comprising the steps of:
a) initially enriching the proportion of fetal nucleated erythrocytes present in the sample of blood by separating non-nucleated erythrocytes from nucleated erythrocytes present in the sample on the basis of size and density, to produce an enriched maternal sample;
b) further enriching the proportion of fetal nucleated erythrocytes present in the enriched maternal sample by combining the enriched maternal sample with at least one detectable monoclonal antibody selective for fetal nucleated erythrocytes, under conditions appropriate for binding of detectable monoclonal antibody with fetal nucleated erythrocytes, to produce bound detectable monoclonal antibody; and
c) separating bound detectable monoclonal antibody from the sample.

15. The method of Claim 14 further comprising,
a) in step (b), combining the enriched maternal sample with at least two detectable monoclonal antibodies, wherein the first of said detectable monoclonal antibodies is selective for antigens present on mature human leucocytes and for antigens present on very immature erythrocyte precursors, but not for mature nucleated erythrocytes; the second of said detectable monoclonal antibodies is selective for an antigen which is present on the surface of fetal nucleated erythrocytes, but not for antigens present on mature human leucocytes or antigens present on very immature erythrocytes; and the two detectable monoclonal antibodies are separately detectable, to produce a first bound detectable monoclonal antibody and a second bound detectable monoclonal antibody; and
b) in step (c), separating the first bound detectable monoclonal antibody and the second bound detectable monoclonal antibody from one another; and for example wherein the first of said detectable monoclonal antibodies is selected from the group consisting of HLe-1, L4 and M3 HLe-1 and the second of said detectable monoclonal antibodies is anti-TfR antibody.

16. A method of detecting the occurrence of fetal DNA of interest in fetal DNA in a sample of blood obtained from a pregnant woman, comprising the steps of:
a) separating fetal nucleated cells present in the sample of peripheral blood from the sample, to produce separated fetal nucleated cells;
b) treating separated fetal nucleated cells to render DNA present in said cells available for hybridization with a complementary nucleotide sequence;
c) contacting the product of step (b) with a selected DNA probe which is a nucleotide sequence complementary to the fetal DNA of interest, under conditions appropriate for hybridization of complementary DNA sequences to occur; and
d) detecting hybridization between the product of step (b) and the selected DNA probe, the occurrence of hybridization being indicative of the presence of fetal DNA of interest in the fetal DNA.

17. A method of detecting the occurrence of a fetal DNA of interest in fetal DNA in a sample of peripheral blood obtained from a pregnant woman, comprising the steps of:
a) separating fetal nucleated erythrocytes present in the sample of peripheral blood from the sample;
b) amplifying DNA present in the separated fetal nucleated erythrocytes, to produce amplified fetal DNA;
c) treating amplified fetal DNA to render it available for hybridization with a complementary nucleotide sequence;
d) combining the product of step (c) with a DNA probe which is a nucleotide sequence complementary to the fetal DNA of interest; and
e) detecting hybridization between the product of step (c) and the DNA probe, the occurrence of hybridization being indicative of the presence of fetal DNA of interest in the fetal DNA.

18. A kit for detecting fetal DNA of interest in a sample of maternal blood comprising:
a) at least one antibody which is selective for a surface antigen characteristic of fetal nucleated cells but not selective for a surface antigen characteristic of maternal leucocytes;
b) selected DNA primers; and
c) at least one DNA probe complementary to the fetal DNA of interest.

19. A kit for detecting fetal DNA of interest in a sample of maternal blood comprising:
a) at least one antibody which is selective for a surface antigen characteristic of fetal nucleated cells but not selective for a surface antigen characteristic of maternal leucocytes;
b) a solid support having affixed thereon antibodies selective for the antibody of (a);
c) selected DNA primers; and
d) at least one DNA probe complementary to the selected DNA.

20. The kit of Claim 19, wherein the solid support is a magnetic bead and further Comprising a magnet.

21. A kit for separating fetal nucleated cells present in a sample of blood obtained from a pregnant woman comprising:
a) at least one antibody which is selective for a surface antigen characteristic of fetal nucleated cells but not selective for a surface antigen characteristic of maternal cells; and
b) a solid support having affixed thereon antibodies selective for the antibody of (a).
